(19) Europäisches Patentamt
European Patent Office
Office européen des brevets

(11) EP 1 284 259 A1

(12) # EUROPEAN PATENT APPLICATION
published in accordance with Art. 158(3) EPC

(43) Date of publication:
**19.02.2003 Bulletin 2003/08**

(21) Application number: **01930239.7**

(22) Date of filing: **22.05.2001**

(51) Int Cl.7: **C07C 401/00**, A61K 31/593, A61P 3/02

(86) International application number:
**PCT/JP01/04256**

(87) International publication number:
**WO 01/090061 (29.11.2001 Gazette 2001/48)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **23.05.2000 JP 2000151298**

(71) Applicant: **CHUGAI SEIYAKU KABUSHIKI KAISHA**
**Tokyo, 115-8543 (JP)**

(72) Inventors:
- **TAKAYAMA, Hiroaki**
**Shibuya-ku, Tokyo 151-0072 (JP)**
- **FUJISHIMA, Toshie**
**Hachioji-shi, Tokyo 193-0834 (JP)**

(74) Representative: **HOFFMANN - EITLE**
**Patent- und Rechtsanwälte**
**Arabellastrasse 4**
**81925 München (DE)**

(54) **5,6- i TRANS /i -2-ALKYLVITAMIN D DERIVATIVES**

(57) Object of the present invention is to synthesize novel vitamin D derivatives.

The present invention provides 5,6-trans-2-alkyl-substituted vitamin D derivatives of Formula (1):

wherein

$R_1$ is straight or branched-chain alkyl; and

$R_2$ is straight or branched-chain alkyl optionally substituted with hydroxy.

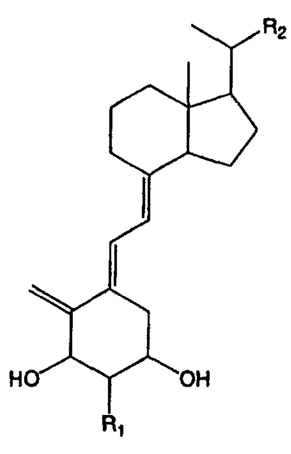



Printed by Jouve, 75001 PARIS (FR)

## Description

### TECHNICAL FIELD

The present invention relates to novel vitamin D derivatives, more particularly, relates to 5,6-trans-2-alkyl-substituted vitamin D derivatives.

### BACKGROUND ART

Activated vitamin $D_3$ derivatives including la,25-dihydroxyvitamin $D_3$ are known to have many physiological activities such as calcium metabolism regulatory activities, growth inhibitory, and differentiation inducing activities for tumor cells, and immunoregulatory activities. However, some activated vitamin $D_3$ derivatives may cause hypercalcemia during long-term and continuous administration, therefore they are not suitable for use as antitumor agents, antirheumatic agents, and the like. Thus, a number of studies have been conducted to synthesize vitamin D derivatives for the purpose of separating those activities.
The studies conducted by the inventors of the present invention clarified that introduction of a 2$\alpha$-methyl group into an A ring part of active vitamin $D_3$, that is 1$\alpha$,25-dihydroxyvitamin $D_3$, increases the vitamin D receptor (VDR) binding property (Bioorg. Med. Chem. Lett., 1998, 8, 151; K. Konno et al.). Furthermore, a combination of the introduction of a 2$\alpha$-methyl group and the epimerization of the side chain at 20-position has been reported to enhance the VDR binding property (Bioorg. Med. Chem. Lett., 1998, 8, 2145; T. Fujishima et al.). However, no work has been done to synthesize a vitamin D derivative in which the 2-position is substituted, the steric configuration at the 20-position is native or epimerized, and the double bond at the 5-position is in E configuration; further, the physiologically activities of such a vitamin D derivative have not been studied.

### DISCLOSURE OF THE INVENTION

To provide vitamin $D_3$ derivatives in which the above problems are improved, the inventors of the present invention intensively studied vitamin $D_3$ derivatives, in which the 2-position is substituted, the steric configuration at the 20-position is native or epimerized and the double bond at the 5-position is in E configuration.
As a result of careful studies to solve the above problems, the inventors have found that the stated object could be achieved by providing vitamin $D_3$ derivatives, in which the 2-position is substituted with alkyl and the double bond at the 5-position is in E configuration, and thereby completed the present invention.
According to the present invention, there is provided a 5,6-trans-2-alkyl-substituted vitamin D derivative of Formula (I):

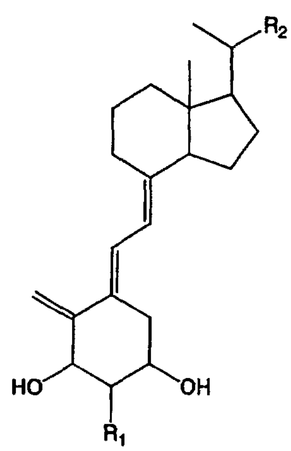

wherein

$R_1$ is straight or branched-chain alkyl and $R_2$ is straight or branched-chain alkyl optionally substituted with hydroxy.
Preferably, $R_1$ is straight or branched-chain $C_{1-6}$alkyl and $R_2$ is straight or branched-chain $C_{1-12}$alkyl substituted with hydroxy in Formula (1).
More preferably, $R_1$ is straight or branched-chain $C_{1-3}$alkyl and $R_2$ is straight or branched-chain $C_{3-10}$alkyl substituted with hydroxy.

Still more preferably, $R_1$ is methyl or ethyl and $R_2$ is 4-hydroxy-4-methylpentyl or 4-ethyl-4-hydroxyhexyl.
Most preferably, $R_1$ is methyl and $R_2$ is 4-hydroxy-4-methylpentyl.
The steric configuration at the 20-position of the compound of Formula (1) may be either S or R.

PREFERRED MODE FOR CARRYING OUT THE INVENTION

Detailed mode and specific examples for carrying out the vitamin D derivatives of Formula (1) of the present invention will be explained below.
In Formula (1), $R_1$ is straight or branched-chain alkyl. $R_2$ is straight or branched-chain alkyl optionally substituted with hydroxy.
As used herein, generally, the straight or branched-chain alkyl is preferably straight or branched-chain lower alkyl. The straight or branched-chain lower alkyl generally means straight or branched-chain $C_{1-15}$alkyl; examples thereof include methyl, ethyl, n-propyl, i-propyl, n-butyl, s-butyl, i-butyl, and t-butyl as well as pentyl, hexyl, heptyl, octyl, nonyl, and decanyl.
The straight or branched-chain alkyl substituted with hydroxy means that at least one hydrogen atom of the above-mentioned alkyl is substituted with hydroxy. In the definition of $R_2$, the number of hydrogen atoms substituted with hydroxy is 1, 2, or 3, preferably 1 or 2 and more preferably 1.
Non-limiting examples of $R_1$ include methyl, ethyl, propyl, butyl, pentyl, hexyl, heptyl, octyl, nonyl, decanyl, and the like. Preferably $R_1$ is straight or branched-chain $C_{1-6}$alkyl, more preferably straight or branched-chain $C_{2-4}$alkyl, still more preferably methyl or ethyl and most preferably methyl.
Non-limiting examples of $R_2$ include 4-hydroxy-4-methylpentyl, 4-ethyl-4-hydroxyhexyl, 6-hydroxy-6-methyl-2-heptyl, 7-hydroxy-7-methyl-2-octyl, 5,6-dihydroxy-6-methyl-2-heptyl, 4,6,7-trihydroxy-6-methyl-2-heptyl, and the like.
Preferably $R_2$ is straight or branched-chain $C_{1-12}$alkyl substituted with hydroxy, more preferably straight or branched-chain $C_{3-10}$alkyl substituted with hydroxy, still more preferably 4-hydroxy-4-methylpentyl or 4-ethyl-4-hydroxyhexyl and most preferably 4-hydroxy-4-methylpentyl.
The vitamin D derivatives of Formula (1) of the present invention can be used as active ingredients of pharmaceutical compositions (such as a calcium metabolism regulating agent). They can also be used as regents for investigating metabolism of active vitamin $D_3$ (i.e., 1$\alpha$,25-dihydroxyvitamin $D_3$).
Although there is no limitation with respect to methods of synthesizing the vitamin D derivatives of Formula (I) of the present invention which are the novel compounds, they can be synthesized, for example, according to synthesis route shown in the following Examples. In the following Examples, vitamin D derivatives of the present invention, which are in a trans form, are synthesized from Compound D, which is in a cis form, according to the following reaction scheme.

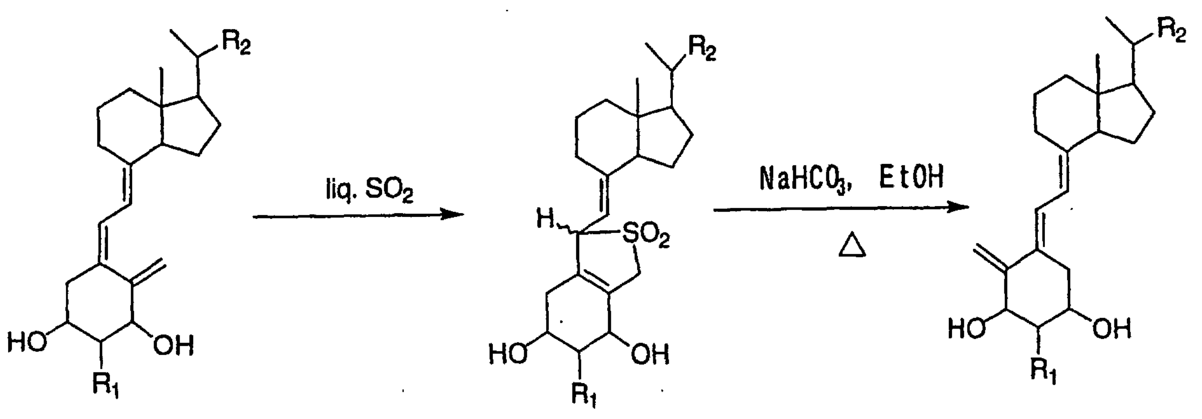


D

Trans-D

Compound D (cis form) in the above reaction scheme is known and can be synthesized according to the methods described in, for example, JP 6-23185 B, JP 6-41059 A, JP 11-116551 A, and JP 11-121589 A (filed by the same applicant as that of the present application).
Contents of the specification of Japanese Patent Application No. 2000-151298, the application on the basis of which the present application claims priority, are to be incorporated in their entirety by reference.
The present invention will be described specifically by way of the following Examples, which in no way limit the invention.

## EXAMPLES

(Test example) Assay for binding to bovine thymus vitamin D receptor (VDR)

Ethanol solutions of 1α,25-dihydroxyvitamin $D_3$ (the standard substance) and those of the vitamin D derivatives of the present invention were prepared at various concentrations. Bovine thymus 1α,25-dihydroxyvitamin $D_3$ receptor was purchased from Yamasa Biochemcal (Choshi, Chiba, Japan) (lot.111031 and lot.112831) and one ampule (approximately 25 mg) of the receptor was dissolved in 55 mL of 0.05 M phosphate 0.5M potassium buffer (pH 7.4) just before use.

Each of the ethanol solutions (50 μl) of vitamin D derivatives of the present invention and 1α,25-dihydroxyvitamin $D_3$ was put into a respective tube with 500 μl (0.23 mg protein) of the receptor solution, preincubated at room temperature for 1 hour, and [$^3$H]-1α,25-dihydroxyvitamin $D_3$ was added at the final concentration of 0.1 nM, followed by incubation overnight at 4°C. Each of the reaction mixtures was mixed with DCC (dextran coated charcoal), left for 30 minutes at 4°C and centrifuged at 3000 rpm for ten minutes to separate the bound and free forms of [$^3$H]-1α,25-dihydroxyvitamin $D_3$. Each of the resultant supernatants (500 μl) was mixed with ACS-II (9.5 ml) (AMERSHAM, England) for radioactivity measurement.

(5E,7E)-(1S,2R,3R)-2-Methyl-9,10-seco-5,7,10(19)-cholestatriene-1,3,25-triol, (5E,7E)-(1S,2S,3R)-2-methyl-9,10-seco-5,7,10(19)-cholestatriene-1,3,25-triol, (5E,7E)-(1R,2R,3R)-2-methyl-9,10-seco-5,7,10(19)-cholestatriene-1,3,25-triol, (5E,7E)-(1R,2S,3R)-2-methyl-9,10-seco-5,7,10(19)-cholestatriene-1,3,25-triol, (5E,7E)-(1S,2R,3S)-2-methyl-9,10-seco-5,7,10(19)-cholestatriene-1,3,25-triol, (5E,7E)-(1S,2S,3S)-2-methyl-9,10-seco-5,7,10(19)-cholestatriene-1,3,25-triol, (5E,7E)-(1R,2R,3S)-2-methyl-9,10-seco-5,7,10(19)-cholestatriene-1,3,25-triol, (5E,7E)-(1R,2S,3S)-2-methyl-9,10-seco-5,7,10(19)-cholestatriene-1,3,25-triol, (5E,7E)-(1S,2R,3R,20S)-2-methyl-9,10-seco-5,7,10(19)-cholestatriene-1,3,25-triol, (5E,7E)-(1S,2S,3R,20S)-2-methyl-9,10-seco-5,7,10(19)-cholestatriene-1,3,25-triol, (5E,7E)-(1R,2R,3R,20S)-2-methyl-9,10-seco-5,7,10(19)-cholestatriene-1,3,25-triol, (5E,7E)-(1R,2S,3R,20S)-2-methyl-9,10-seco-5,7,10(19)-cholestatriene-1,3,25-triol, (5E,7E)-(1S,2R,3S,20S)-2-methyl-9,10-seco-5,7,10(19)-cholestatriene-1,3,25-triol, (5E,7E)-(1S,2S,3S,20S)-2-methyl-9,10-seco-5,7,10(19)-cholestatriene-1,3,25-triol, (5E,7E)-(1R,2R,3S,20S)-2-methyl-9,10-seco-5,7,10(19)-cholestatriene-1,3,25-triol and (5E,7E)-(1R,2S,3S,20S)-2-methyl-9,10-seco-5,7,10(19)-cholestatriene-1,3,25-triol, which were synthesizable in the following Examples 1-16, were used as the vitamin D derivatives of the present invention.

The binding property of the vitamin D derivatives of the present invention expressed in relative value with that of 1α,25-dihydroxyvitamin $D_3$ taken as 100 was obtained according to the following equation and the values calculated are shown in the respective Examples, after the physical data of the respective derivatives.

$$X=(y/x)\times 100$$

X: relative VDR binding property of the vitamin D derivatives of the present invention
y: concentration of 1α,25-dihydroxyvitamin $D_3$ that inhibits 50% of the binding of [$^3$H]-1α,25-dihydroxyvitamin $D_3$ and VDR
x: concentration of the vitamin D derivatives of the present invention that inhibits 50% of the binding of [$^3$H]-1α,25-dihydroxyvitamin $D_3$ and VDR

(Example 1)

Synthesis of (5E,7E)-(1S,2R,3R)-2-methyl-9,10-seco-5,7,10(19)-cholestatriene-1,3,25-triol (Compound (t-Aa))

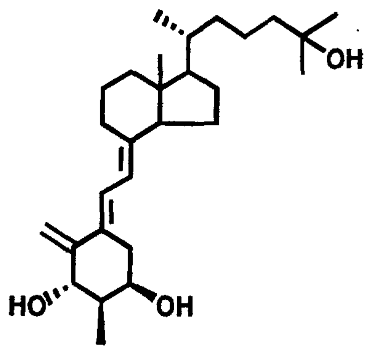

t-Aa

(5Z,7E)-(1S,2R,3R)-2-Methyl-9,10-seco-5,7,10(19)-cholestatriene-1,3,25-triol (Compound (Aa)) (7.0 mg, 0.016 mmol) was dissolved in liquid sulfur dioxide (~10 mL). This solution was refluxed under heating at the boiling point of the liquid sulfur dioxide for 1 hour. After distilling off the liquid sulfur dioxide, the resulting residue was dissolved in ethanol (2 mL), to which sodium hydrogen carbonate (6.8 mg, 0.081 mmol) was added. The mixture was heated at 90°C for 1 hour. After distilling off the solvent, the residue was mixed with water and extracted with ethyl acetate. The organic layer was dried over magnesium sulfate, filtered and the filtrate was concentrated. Thus obtained crude product was purified by silica gel preparative thin layer chromatography to give Compound (t-Aa) (4.6 mg, 66%) as a colorless oil.
UV (EtOH) λmax 272 nm, λmin 230 nm; $^1H$ NMR (400 MHz, $CDCl_3$) δ 0.56 (3 H, s), 0.94 (3 H, d, J = 6.4 Hz), 1.08 (3 H, d, J = 7.0 Hz), 1.22 (6 H, s), 1.94 (1 H, m), 2.56 (1 H, dd, J = 13.7, 3.4 Hz), 2.60 (1 H, dd, J = 14.6, 6.7 Hz), 2.83 (1 H, m), 4.13 (1 H, m), 4.14 (1 H, m), 5.01 (1 H, s), 5.15 (1 H, s), 5.87 (1 H, d, J = 11.6 Hz), 6.61 (1 H, d, J = 11.6 Hz); MS 430 $[M]^+$, 412 $[M-H_2O]^+$, 394 $[M-2H_2O]^+$, 379 $[M-2H_2O-Me]^+$; HRMS calcd. for $[C_{28}H_{46}O_3]$ 430.3447, found 430.3446. VDR binding property : 8.6

(Example 2)

Synthesis of (5E,7E)-(1S,2S,3R)-2-methyl-9,10-seco-5,7,10(19)-cholestatriene-1,3,25-triol (Compound (t-Ds))

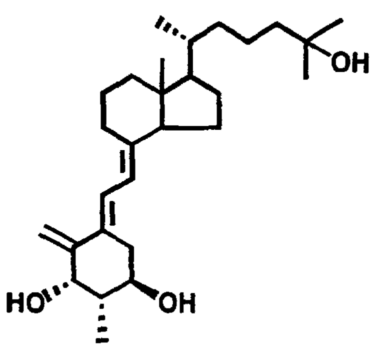

t-Ds

The title compound (t-Ds) was synthesized from the corresponding (5Z)-isomer of the title compound (t-Ds) according to the same procedure as Example 1. UV (EtOH) λmax 273 nm, λmin 230 nm; $^1$H NMR (400 MHz, $CDCl_3$) δ 0.57 (3 H, s), 0.95 (3 H, d, J = 6.4 Hz), 1.14 (3 H, d, J = 7.0 Hz), 1.22 (6 H, s), 1.83 (1 H, m), 2.13 (1 H, m), 2.85 (1 H, m), 3.02 (1 H, dd, J = 14.0, 4.3 Hz), 3.85 (1 H, m), 4.29 (1 H, m), 4.93 (1 H, s), 5.12 (1 H, d, J = 1.8 Hz), 5.89 (1 H, d, J = 11.6 Hz), 6.55 (1 H, dd, J = 11.6, 0.9 Hz); MS 430 $[M]^+$, 412 $[M-H_2O]^+$, 394 $[M-2H_2O]^+$, 379 [M-$2H_2O$-Me]+; HRMS calcd. for $[C_{28}H_{46}O_3]$ 430.3447, found 430.3447. VDR binding property :0.4

(Example 3)

Synthesis of (5E,7E)-(1R,2R,3R)-2-methyl-9,10-seco-5,7,10(19)-cholestatriene-1,3,25-triol (Compound (t-As))

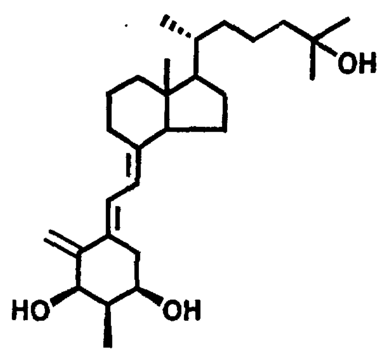

**t-As**

The title compound (t-As) was synthesized from the corresponding (5Z)-isomer of the title compound (t-As) according to the same procedure as Example 1. UV (EtOH) λmax 274 nm, λmin 231 nm; $^1$H NMR (400 MHz, $CDCl_3$) δ 0.57 (3 H, s), 0.94 (3 H, d, J = 6.4 Hz), 1.22 (6 H, s), 1.24 (3 H, d, J = 7.0 Hz); 1.92 (1 H, ddq, J = 2.4, 2.5, 7.0 Hz), 2.27 (1 H, dd, J = 14.7, 3.1 Hz), 2.88 (1 H, dd, J = 12.8, 3.7 Hz), 3.05 (1 H, dd, J = 14.6, 3.7 Hz), 3.97 (1 H, ddd, J = 2.4, 3.1, 3.7 Hz), 4.21 (1 H, d, J = 2.5 Hz), 4.90 (1 H, d, J = 1.8 Hz), 5.10 (1 H, d, J = 1.8 Hz), 5.91 (1 H, d, J = 11.3 Hz), 6.67 (1 H, d, J = 11.3 Hz); MS 430 $[M]^+$, 412 $[M-H_2O]^+$, 394 $[M-2H_2O]^+$, 379 $[M-2H_2O-Me]^+$; HRMS calcd. for $[C_{28}H_{46}O_3]$ 430.3447, found 430.3449.
VDR binding property :0.1

(Example 4)

Synthesis of (5E,7E)-(1R,2S,3R)-2-methyl-9,10-seco-5,7,10(19)-cholestatriene-1,3,25-triol (Compound (t-Da))

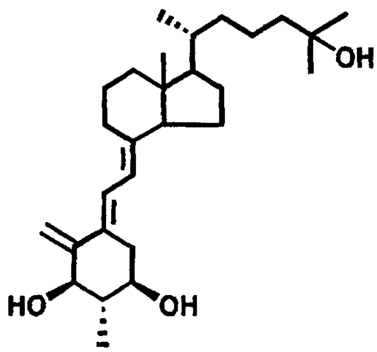

**t-Da**

The title compound (t-Da) was synthesized from the corresponding (5Z)-isomer of the title compound (t-Da) according to the same procedure as Example 1. UV (EtOH) λmax 271 nm, λmin 229 nm; $^1$H NMR (400 MHz, $CDCl_3$) δ 0.57 (3 H, s), 0.95 (3 H, d, J = 6.2 Hz), 1.03 (3 H, d, J = 7.0 Hz), 1.22 (6 H, s), 1.89 (1 H, ddq, J = 5.5, 4.8, 7.0 Hz), 2.06 (1 H, dd, J = 15.0, 5.8 Hz), 2.65 (1 H, dd, J = 15.0, 4.8 Hz), 2.87 (1 H, dd, J = 12.2, 3.7 Hz), 3.71 (1 H, dt, J = 5.8, 4.8 Hz), 3.98 (1 H, d, J = 5.5 Hz), 4.97 (1 H, s), 5.17 (1 H, s), 5.89 (1 H, d, J = 11.7 Hz), 6.62 (1 H, d, J = 11.7 Hz); MS 430 $[M]^+$, 412 $[M-H_2O]^+$, 394 $[M-2H_2O]^+$, 379 $[M-2H_2O-Me]^+$; HRMS calcd. for $[C_{28}H_{46}O_3]$ 430.3447, found 430.3448. VDR binding property :<0.01

(Example 5)

Synthesis of (5E,7E)-(1S,2R,3S)-2-methyl-9,10-seco-5,7,10(19)-cholestatriene-1,3,25-triol (Compound (t-Ba))

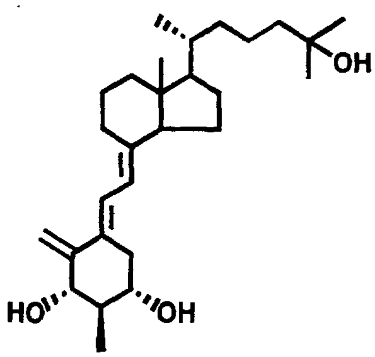

**t-Ba**

The title compound (t-Ba) was synthesized from the corresponding (5Z)-isomer of the title compound (t-Ba) according to the same procedure as Example 1.
UV (EtOH) λmax 271 nm, λmin 229 nm; $^1$H NMR (400 MHz, $CDCl_3$) δ 0.57 (3 H, s), 0.95 (3 H, d, J = 6.4 Hz), 1.03 (3 H, d, J = 7.0 Hz), 1.22 (6 H, s), 1.91 (1 H, ddq, J = 5.1, 4.8, 7.0 Hz), 2.61 (1 H, dd, J = 15.0, 4.4 Hz), 2.65 (1 H, dd, J = 15.0, 5.1 Hz), 2.86 (1 H, dd, J = 11.9, 3.7 Hz), 3.74 (1 H, dt, J = 4.4, 5.1 Hz), 4.00 (1 H, d, J = 5.1 Hz), 4.97 (1 H, s), 5.18 (1 H, d, J = 1.6 Hz), 5.90 (1 H, d, J = 11.6 Hz), 6.62 (1 H, d, J = 11.6 Hz); MS 430 $[M]^+$, 412 $[M-H_2O]^+$, 394 [M-

$2H_2O]^+$, 379 $[M-2H_2O-Me]^+$; HRMS calcd. for $[C_{28}H_{46}O_3]$ 430.3447, found 430.3444.
VDR binding property :0.04

(Example 6)

Synthesis of (5E,7E)-(1S,2S,3S)-2-methyl-9,10-seco-5,7,10(19)-cholestatriene-1,3,25-triol (Compound (t-Cs))

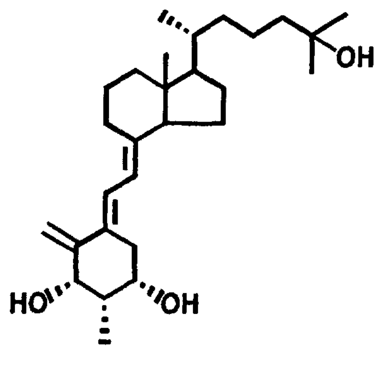

**t-Cs**

The title compound (t-Cs) was synthesized from the corresponding (5Z)-isomer of the title compound (t-Cs) according to the same procedure as Example 1.
UV (EtOH) λmax 274 nm, λmin 231 nm; $^1H$ NMR (400 MHz, $CDCl_3$) δ 0.57 (3 H, s), 0.95 (3 H, d, J = 6.4 Hz), 1.22 (6 H, s), 1.24 (3 H, d, J = 7.0 Hz), 1.93 (1 H, ddq, J = 2.4, 2.1, 7.0 Hz), 2.28 (1 H, dd, J = 14.6, 2.4 Hz), 2.88 (1 H, dd, J = 12.2, 3.7 Hz), 3.06 (1 H, dd, J = 14.6, 3.7 Hz), 3.98 (1 H, ddd, J = 3.7, 2.4, 2.1 Hz), 4.21 (1 H, d, J = 2.1 Hz), 4.91 (1 H, d, J = 1.8 Hz), 5.12 (1 H, d, J = 1.8 Hz), 5.92 (1 H, d, J = 11.3 Hz), 6.67 (1 H, d, J = 11.3 Hz); MS 430 $[M]^+$, 412 $[M-H_2O]^+$, 394 $[M-2H_2O]^+$, 379 $[M-2H_2O-Me]^+$; HRMS calcd. for $[C_{28}H_{46}O_3]$ 430.3447, found 430.3448.
VDR binding property :0.013

(Example 7)

Synthesis of (5E,7E)-(1R,2R,3S)-2-methyl-9,10-seco-5,7,10(19)-cholestatriene-1,3,25-triol (Compound (t-Bs))

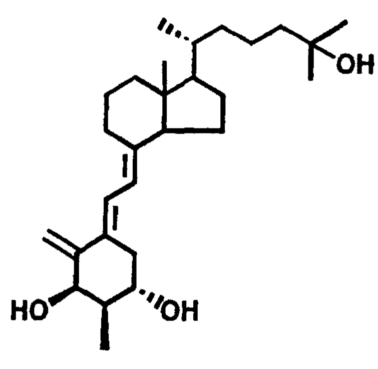

**t-Bs**

The title compound (t-Bs) was synthesized from the corresponding (5Z)-isomer of the title compound (t-Bs) according to the same procedure as Example 1.

UV (EtOH) λmax 275 nm, λmin 231 nm; $^1$H NMR (400 MHz, $CDCl_3$) δ 0.57 (3 H, s), 0.95 (3 H, d, J = 6.4 Hz), 1.14 (3 H, d, J = 7.0 Hz), 1.22 (6 H, s), 1.83 (1 H, ddq, J = 9.2, 3.1, 7.0 Hz), 2.13 (1 H, dd, J = 14.0, 9.2 Hz), 2.85 (1 H, dd, J = 11.9, 4.0 Hz), 3.01 (1 H, dd, J = 14.0, 4.5 Hz), 3.87 (1 H, dt, J = 4.5, 9.2 Hz), 4.30 (1 H, d, J =3.1 Hz), 4.93 (1 H, d, J =1.8 Hz), 5.11 (1 H, d, J =1.8 Hz), 5.89 (1 H, d, J = 11.6 Hz), 6.55 (1 H, d, J = 11.6 Hz); MS 430 $[M]^+$, 412 $[M-H_2O]^+$, 394 $[M-2H_2O]^+$, 379 $[M-2H_2O-Me]^+$.
VDR binding property :0.03

(Example 8)

Synthesis of (5E,7E)-(1R,2S,3S)-2-methyl-9,10-seco-5,7,10(19)-cholestatriene-1,3,25-triol (Compound (t-Ca))

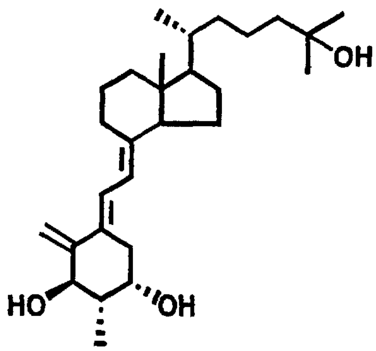

**t-Ca**

The title compound (t-Ca) was synthesized from the corresponding (5Z)-isomer of the title compound (t-Ca) according to the same procedure as Example 1.
UV (EtOH) λmax 274 nm, λmin 232 nm; $^1$H NMR (400 MHz, $CDCl_3$) δ 0.57 (3 H, s), 0.95 (3 H, d, J = 6.4 Hz), 1.08 (3 H, d, J = 7.0 Hz), 1.22 (6 H, s), 1.93 (1 H, ddq, J = 8.0, 3.4, 7.0 Hz), 2.53 (1 H, dd, J = 14.3, 3.4 Hz), 2.61 (1 H, dd, J = 14.3, 5.8 Hz), 2.85 (1 H, dd, J = 12.2, 3.7 Hz), 4.15 (2 H, m), 5.01 (1 H, s), 5.15 (1 H, s), 5.86 (1 H, d, J = 11.3 Hz), 6.60 (1 H, d, J = 11.3 Hz); MS 430 $[M]^+$, 412 $[M-H_2O]^+$, 394 $[M-2H_2O]^+$, 379 $[M-2H_2O-Me]^+$; HRMS calcd. for $[C_{28}H_{46}O_3]$ 430.3447, found 430.3443.
VDR binding property :<0.01

(Example 9)

Synthesis of (5E,7E)-(1S,2R,3R,20S)-2-methyl-9,10-seco-5,7,10(19)-cholestatriene-1,3,25-triol (Compound (20-epi-t-Aa))

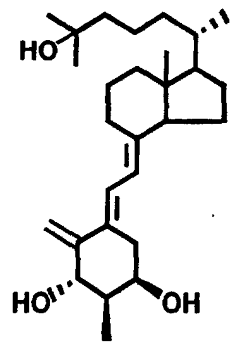

**20-*epi*-t-Aa**

The title compound (20-epi-t-Aa) was synthesized from the corresponding (5Z)-isomer of the title compound (20-epi-t-Aa) according to the same procedure as Example 1.
UV (EtOH) λmax 272 nm, λmin 230 nm; $^1$H NMR (400 MHz, $CDCl_3$) 6 0.56 (3 H, s), 0.86 (3 H, d, J = 6.4 Hz), 1.09 (3 H, d, J = 7.0 Hz), 1.22 (6 H, s), 1.88 (1 H, m), 1.95 (1 H, ddq, J = 7.6, 3.4, 7.0 Hz), 2.53 (1 H, dd, J = 14.3, 4.0 Hz), 2.60 (1 H, dd, J = 14.6, 7.0 Hz), 4.13 (1 H, d, J = 7.6 Hz), 4.17 (1 H, ddd, J = 7.0, 4.0, 3.4 Hz), 5.01 (1 H, s), 5.16 (1 H, s), 5.87 (1 H, d, J = 11.6 Hz), 6.61 (1 H, d, J = 11.6 Hz); MS 430 $[M]^+$, 412 $[M-H_2O]^+$, 394 $[M-2H_2O]^+$, 379 $[M-2H_2O-Me]^+$; HRMS calcd. for $[C_{28}H_{46}O_3]$ 430.3447, found 430.3445.
VDR binding property :45

(Example 10)

Synthesis of (5E,7E)-(1S,2S,3R,20S)-2-methyl-9,10-seco-5,7,10(19)-cholestatriene-1,3,25-triol (Compound (20-epi-t-Ds))

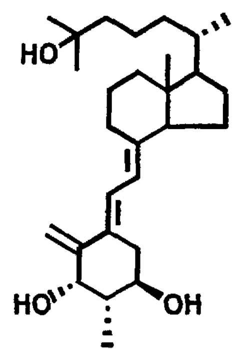

**20-*epi*-t-Ds**

The title compound (20-epi-t-Ds) was synthesized from the corresponding (5Z)-isomer of the title compound (20-epi-t-Ds) according to the same procedure as Example 1.

UV (EtOH) λmax 273 nm, λmin 228 nm; $^1$H NMR (400 MHz, $CDCl_3$) δ 0.56 (3 H, s), 0.86 (3 H, d, J = 6.4 Hz), 1.14 (3 H, d, J = 7.0 Hz), 1.22 (6 H, s), 1.83 (1 H, m), 3.01 (1 H, dd, J = 14.3, 5.2 Hz), 3.84 (1 H, m), 4.29 (1 H, m), 4.93 (1 H, d, J = 2.1 Hz), 5.12 (1 H, d, J = 2.1 Hz), 5.89 (1 H, d, J = 11.4 Hz), 6.54 (1 H, d, J = 11.4 Hz); MS 430 $[M]^+$, 412 $[M-H_2O]^+$, 394 $[M-2H_2O]^+$, 379 $[M-2H_2O-Me]^+$.
VDR binding property :1

(Example 11)

Synthesis of (5E,7E)-(1R,2R,3R,20S)-2-methyl-9,10-seco-5,7,10(19)-cholestatriene-1,3,25-triol (Compound (20-epi-t-As))

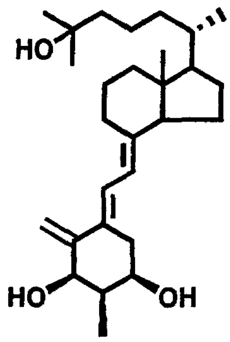

**20-*epi*-t-As**

The title compound (20-epi-t-As) was synthesized from the corresponding (5Z)-isomer of the title compound (20-epi-t-As) according to the same procedure as Example 1.
UV (EtOH) λmax 274 nm, λmin 231 nm; $^1$H NMR (400 MHz, $CDCl_3$) δ 0.57 (3 H, s), 0.86 (3 H, d, J = 6.4 Hz), 1.21 (6 H, s), 1.24 (3 H, d, J = 7.0 Hz), 2.28 (1 H, br. d, J = 14.6 Hz), 2.88 (1 H, dd, J = 12.4, 3.7 Hz), 3.04 (1 H, dd, J = 15.0, 4.0 Hz), 3.97 (1 H, m), 4.21 (1 H, m), 4.90 (1 H, d, J = 1.5 Hz), 5.10 (1 H, d, J = 1.8 Hz), 5.91 (1 H, d, J = 11.6 Hz), 6.66 (1 H, d, J = 11.6 Hz); MS 430 $[M]^+$, 412 $[M-H_2O]^+$, 394 $[M-2H_2O]^+$, 379 $[M-2H_2O-Me]^+$; HRMS calcd. for $[C_{28}H_{46}O_3]$ 430.3447, found 430.3423.
VDR binding property :0.8

(Example 12)

Synthesis of (5E,7E)-(1R,2S,3R,20S)-2-methyl-9,10-seco-5,7,10(19)-cholestatriene-1,3,25-triol (Compound (20-epi-t-Da))

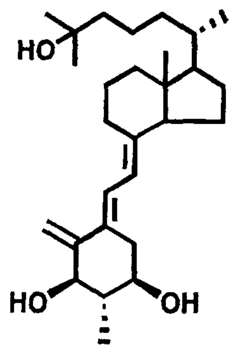

**20-*epi*-t-Da**

The title compound (20-epi-t-Da) was synthesized from the corresponding (5Z)-isomer of the title compound (20-epi-t-Da) according to the same procedure as Example 1.
UV (EtOH) λmax 270 nm, λmin 230 nm; $^1$H NMR (400 MHz, $CDCl_3$) 6 0.57 (3 H, s), 0.86 (3 H, d, J = 6.4 Hz), 1.03 (3 H, d, J = 7.0 Hz), 1.22 (6 H, s), 1.87 (1 H, m), 2.60 (1 H, dd, J = 15.9, 6.1 Hz), 2.64 (1 H, m), 2.87 (1 H, dd, J = 11.9, 4.0 Hz), 3.71 (1 H, m), 3.98 (1 H, m), 4.97 (1 H, s), 5.17 (1 H, d, J = 1.8 Hz), 5.90 (1 H, d, J = 11.6 Hz), 6.61 (1 H, d, J = 11.6 Hz); MS 430 $[M]^+$, 412 $[M-H_2O]^+$, 397 $[M-H_2O-Me]^+$; HRMS calcd. for $[C_{28}H_{46}O_3]$ 430.3447, found 430.3465.
VDR binding property :0.03

(Example 13)

Synthesis of (5E,7E)-(1S,2R,3S,20S)-2-methyl-9,10-seco-5,7,10(19)-cholestatriene-1,3,25-triol (Compound (20-epi-t-Ba))

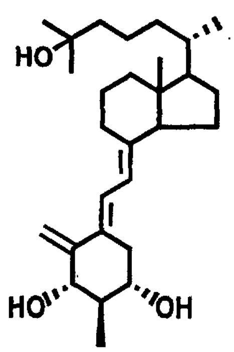

**20-*epi*-t-Ba**

The title compound (20-epi-t-Ba) was synthesized from the corresponding (5Z)-isomer of the title compound (20-epi-t-Ba) according to the same procedure as Example 1.

UV (EtOH) λmax 271 nm, λmin 229 nm; $^1$H NMR (400 MHz, $CDCl_3$) δ 0.56 (3 H, s), 0.86 (3 H, d, J = 6.4 Hz), 1.03 (3 H, d, J = 7.4 Hz), 1.21 (6 H, s), 2.62 (2 H, m), 2.86 (1 H, m), 3.75 (1 H, m), 4.00 (1 H, m), 4.97 (1 H, s), 5.18 (1 H, d, J = 1.8 Hz), 5.90 (1 H, d, J = 11.6 Hz), 6.62 (1 H, d, J = 11.9 Hz); MS 430 $[M]^+$, 412 $[M-H_2O]^+$, 394 $[M-2H_2O]^+$, 379 $[M-2H_2O-Me]^+$; HRMS calcd. for $[C_{28}H_{46}O_3]$ 430.3447, found 430.3442.
VDR binding property :0.5

(Example 14)

Synthesis of (5E,7E)-(1S,2S,3S,20S)-2-methyl-9,10-seco-5,7,10(19)-cholestatriene-1,3,25-triol (Compound (20-epi-t-Cs))

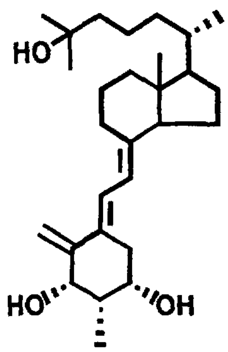

**20-*epi*-t-Cs**

The title compound (20-epi-t-Cs) was synthesized from the corresponding (5Z)-isomer of the title compound (20-epi-t-Cs) according to the same procedure as Example 1.
UV (EtOH) λmax 274 nm, λmin 231 nm; $^1$H NMR (400 MHz, $CDCl_3$) δ 0.57 (3 H, s), 0.86 (3 H, d, J = 6.4 Hz), 1.22 (6 H, s), 1.24 (3 H, d, J = 7.3 Hz), 2.27 (1 H, br. d, J = 14.1 Hz), 2.87 (1 H, dd, J = 12.5, 4.0 Hz), 3.06 (1 H, dd, J = 14.0, 3.7 Hz), 3.98 (1 H, m), 4.20 (1 H,m), 4.91 (1 H, d, J = 1.8 Hz), 5.12 (1 H, d, J = 1.8 Hz), 5.92 (1 H, d, J = 11.6 Hz), 6.66 (1 H, d, J = 11.9 Hz); MS 430 $[M]^+$, 412 $[M-H_2O]^+$, 397 $[M-H_2O-Me]^+$; HRMS calcd. for $[C_{28}H_{46}O_3]$ 430.3447, found 430.3453.
VDR binding property :0.2

(Example 15)

Synthesis of (5E,7E)-(1R,2R,3S,20S)-2-methyl-9,10-seco-5,7,10(19)-cholestatriene-1,3,25-triol (Compound (20-epi-t-Bs))

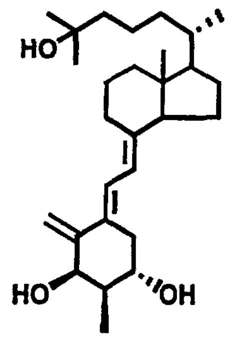

**20-*epi*-t-Bs**

The title compound (20-epi-t-Bs) was synthesized from the corresponding (5Z)-isomer of the title compound (20-epi-t-Bs) according to the same procedure as Example 1.
UV (EtOH) λmax 275 nm, λmin 231 nm; $^1$H NMR (400 MHz, $CDCl_3$) δ 0.57 (3 H, s), 0.86 (3 H, d, J = 6.7 Hz), 1.14 (3 H, d, J = 7.0 Hz), 1.22 (6 H, s), 2.13 (1 H, dd, J = 13.7, 8.5 Hz), 2.85 (1 H, dd, J = 11.9, 4.0 Hz), 3.01 (1 H, dd, J = 14.0, 4.6 Hz), 3.86 (1 H, dt, J = 4.9, 8.5 Hz), 4.29 (1 H, d, J = 2.7 Hz), 4.92 (1 H, d, J =1.2 Hz), 5.10 (1 H, d, J =1.8 Hz), 5.88 (1 H, d, J = 11.6 Hz), 6.55 (1 H, d, J = 11.3 Hz); MS 430 $[M]^+$, 412 $[M-H_2O]^+$, 397 $[M-H_2O-Me]^+$, 379 $[M-2H_2O-Me]^+$; HRMS calcd. for $[C_{28}H_{46}O_3]$ 430.3447, found 430.3444.
VDR binding property :0.2

(Example 16)

Synthesis of (5E,7E)-(1R,2S,3S,20S)-2-methyl-9,10-seco-5,7,10(19)-cholestatriene-1,3,25-triol (Compound (20-epi-t-Ca))

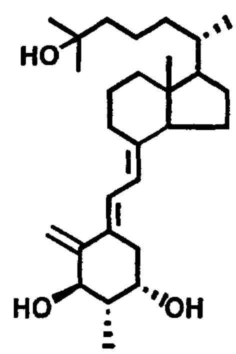

**20-*epi*-t-Ca**

The title compound (20-epi-t-Ca) was synthesized from the corresponding (5Z)-isomer of the title compound (20-epi-t-Ca) according to the same procedure as Example 1.

UV (EtOH) λmax 274 nm, λmin 231 nm; $^1$H NMR (400 MHz, $CDCl_3$) δ 0.56 (3 H, s), 0.86 (3 H, d, J = 6.4 Hz), 1.08 (3 H, d, J = 7.0 Hz), 1.21 (6 H, s), 2.53 (1 H, dd, J = 14.3, 3.1 Hz), 2.60 (1 H, dd, J = 14.3, 5.8 Hz), 2.85 (1 H, dd, J = 12.5, 4.3 Hz), 4.16 (2 H, m), 5.01 (1 H, d, J = 1.2 Hz), 5.15 (1 H, d, J = 1.2 Hz), 5.86 (1 H, d, J = 11.6 Hz), 6.60 (1 H, d, J = 11.3 Hz) ; MS 430 $[M]^+$, 412 $[M-H_2O]^+$, 397 $[M-H_2O-Me]^+$, 379 $[M-2H_2O-Me]^+$; HRMS calcd. for $[C_{28}H_{46}O_3]$ 430.3447, found 430.3446.
VDR binding property :0.08

INDUSTRIAL APPLICABILITY

The vitamin D derivatives represented by Formula (I) are novel and expected to be useful as medicines, for example, for calcium metabolism regulation.

## Claims

1. A 5,6-trans-2-alkyl-substituted vitamin D derivative of Formula (1):

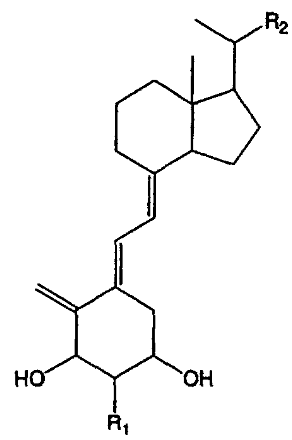

wherein
$R_1$ is straight or branched-chain alkyl; and
$R_2$ is straight or branched-chain alkyl optionally substituted with hydroxy.

2. The vitamin D derivative of claim 1, wherein $R_1$ is straight or branched-chain $C_{1-6}$alkyl and $R_2$ is straight or branched-chain $C_{1-12}$alkyl substituted with hydroxy.

3. The vitamin D derivative of claim 1, wherein $R_1$ is straight or branched-chain $C_{1-3}$alkyl and $R_2$ is straight or branched-chain $C_{3-10}$alkyl substituted with hydroxy.

4. The vitamin D derivative of claim 1, wherein $R_1$ is methyl or ethyl and $R_2$ is 4-hydroxy-4-methylpentyl or 4-ethyl-4-hydroxyhexyl.

5. The vitamin D derivative of claim 4, wherein $R_1$ is methyl and $R_2$ is 4-hydroxy-4-methylpentyl.

6. The vitamin D derivative of claim 1, wherein the stereochemistry at the 20-position is S configuration.

7. The vitamin D derivative of claim 1, wherein the stereochemistry at the 20-position is R configuration.

## INTERNATIONAL SEARCH REPORT

International application No.
PCT/JP01/04256

A. CLASSIFICATION OF SUBJECT MATTER
Int.Cl[7] C07C401/00, A61K31/593, A61P3/02

According to International Patent Classification (IPC) or to both national classification and IPC

B. FIELDS SEARCHED

Minimum documentation searched (classification system followed by classification symbols)
Int.Cl[7] C07C401/00, A61K31/00, A61P3/00

Documentation searched other than minimum documentation to the extent that such documents are included in the fields searched

Electronic data base consulted during the international search (name of data base and, where practicable, search terms used)
REGISTRY (STN), CA (STN)

C. DOCUMENTS CONSIDERED TO BE RELEVANT

| Category* | Citation of document, with indication, where appropriate, of the relevant passages | Relevant to claim No. |
|---|---|---|
| Y | WO 86/06255 A1 (Wisconsin Alumni Research Foundation), 06 November, 1986 (06.11.86), & JP 62-502545 A & US 4800198 A | 1-7 |
| Y | WO 85/01291 A1 (Wisconsin Alumni Research Foundation), 28 March, 1985 (28.03.85), & JP 60-502203 A & US 4512925 A | 1-7 |
| Y | JP 6-41059 A (Chugai Pharmaceutical Co., Ltd.), 15 February, 1994 (15.02.94), pages 2 to 3 (Family: none) | 1-7 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 14 August, 2001 (14.08.01) | 28 August, 2001 (28.08.01) |
| Name and mailing address of the ISA/ Japanese Patent Office | Authorized officer |
| Facsimile No. | Telephone No. |

Form PCT/ISA/210 (second sheet) (July 1992)